# EUROPÄISCHE PATENTANMELDUNG

(11) **EP 0 866 124 A2**
(43) Veröffentlichungstag der Anmeldung: **23.09.1998**
(21) Anmeldenummer: 98105122.0
(22) Anmeldetag: 20.03.1998
(51) Int. Cl.: C12N 15/11, A61K 31/70, C07K 14/705, A61K 38/17, C07K 16/28, A61K 39/395

(54) **Zusammensetzung, enthaltend eine die Aktivität des endogenen T1-M-Proteins direkt oder indirekt modifizierende Verbindung**

(30) Priorität: 21.03.1997 DE 19711933
(71) Anmelder: GSF-Forschungszentrum für Umwelt und Gesundheit GmbH, 85764 Oberschleissheim (DE)
(72) Erfinder: Werenskiold, Anne Katrin, 81377 München (DE); Schmidt, Jörg, 80809 München (DE)
(74) Vertreter: Grünecker, Kinkeldey, Stockmair & Schwanhäusser Anwaltssozietät

(57) **Zusammenfassung**

Die Anmeldung betrifft Zusammensetzungen, die eine Verbindung enthalten, die die Aktivität des T1-M-Proteins moduliert, die Verwendung von Verbindungen, die die Aktivität des T1-M-Proteins modulieren sowie ein in vitro-Verfahren zum Beeinflussen des Proliferations- und/oder Differenzierungspotentials von Knochenzellen.

Die Knochendifferenzierung ist ein komplexer Prozeß, bei dem die Proliferation von Osteoblasten und die Ausbildung von Knochenmatrix eine entscheidende Rolle spielen. Die den Übergang von proliferierenden Osteoblasten in ruhende Knochenzellen induzierenden Signale sind bis heute nicht identifiziert. Die Kenntnis dieser Signale könnte die Behandlung osteopetrotischer und osteoporotischer Erkrankungen ermöglichen.

Erfindungsgemäß wurde festgestellt, daß die Inhibition des T1-M-Proteins auf Osteoblasten proliferationsfördernd wirkt, während seine Aktivierung die Differenzierung anregt. Es werden daher Zusammensetzungen zur Verfügung gestellt, die Verbindungen enthalten, die die Aktivität des T1-M-Proteines modulieren können.

## Beschreibung

### I. Einleitung

Die vorliegende Erfindung betrifft Zusammensetzungen, die eine Verbindung enthalten, die die Aktivität des T1-M-Proteins moduliert, die Verwendung von Verbindungen, die die Aktivität von T1-M-Protein modulieren, sowie ein in vitro-Verfahren zum Beeinflussen des Proliferations- und/oder Differenzierungspotentials von Knochenzellen.

Die Knochendifferenzierung ist ein komplexer Prozeß, der die Proliferation von Osteoblasten und die Ausbildung von Knochenmatrix einbezieht. Während der Kindheit findet ein ausgeprägter Knochenumbau statt, um das Skelettwachstum zu ermöglichen, und dieser Knochenumbau hält während des ganzen Lebens an. Zur Erhaltung einer bestimmten Knochenmasse ist ein ausgewogenes Gleichgewicht zwischen Knochenabbau und Knochenneubildung unerläßlich. Eine verringerte Fähigkeit, neuen Knochen zu bilden, führt zu einem Gesamtverlust an Knochenmasse, der Osteoporose, die vor allem bei Frauen nach der Menopause ein großes medizinisches Problem darstellt.

Die Osteogenese in vivo ebenso wie in verschiedenen Gewebekultursystemen kann in drei aufeinanderfolgende Stadien aufgeteilt werden. Das frühe Proliferationsstadium ist durch DNA-Synthese und die Expression von Genen gekennzeichnet, deren Genprodukte bei der Ausbildung der extrazellulären Matrix eine Rolle spielen, einschließlich von Collagen Typ 1, Fibronektin und TGF-β. Das zweite Stadium der Matrixreifung ist durch eine hohe Expression der alkalischen Phosphatase gekennzeichnet, die die Osteoidmatrix für das dritte Stadium, das durch die Synthese von Osteokalzin gekennzeichnet ist, vorbereiten könnte.

Die Knochendifferenzierungsprozesse werden durch eine große Anzahl von Wachstumsfaktoren und Cytokinen vermittelt, von denen viele in der kalzifizierten Knochenmatrix akkumulieren. Ein Schlüsselereignis, das das Ausmaß der neuen Knochenbildung steuert, ist der Übergang von proliferierenden Osteoblasten in ruhende Knochenzellen, die die Knochenmatrix abgeben. Die diesen Reifungsprozeß induzierenden Signale sind bis heute nicht identifiziert und werden vage als in die Knochenmatrixreifung involvierte Faktoren bezeichnet. Die Kenntnis dieser Faktoren würde es ermöglichen, osteopetrotisches Wachstum zu verringern oder gar völlig zu inhibieren oder umgekehrt Präosteoblasten zur Proliferation anzuregen, um so beispielsweise osteoporotische Krankheitsbilder abzumildern.

Aufgabe der Erfindung ist es daher, Mittel zu finden, mit denen die Differenzierung bzw. die Proliferation von Knochenzellen beeinflußt werden kann.

Erfindungsgemäß wird diese Aufgabe gelöst durch eine Zusammensetzung, die eine Verbindung enthält, die die Aktivität des T1-M-Proteines modulieren kann. Überraschenderweise konnte nämlich gezeigt werden, daß die Inhibition des T1-M-Proteins proliferationsfördernd wirkt, während seine Aktivierung die Differenzierung anregt.

T1-M ist ein Rezeptor, der mit dem Interleukin 1 (IL-1)-Rezeptor nahe verwandt ist, aber keine Affinität für den Liganden IL-1 aufweist. Der an T1-M bindende Ligand ist bis heute nicht genau bekannt, jedoch sind kürzlich zwei mögliche Liganden beschrieben worden (Kumar, 1995; Gayle, 1996). Die biologischen Funktionen dieser Liganden müssen noch geklärt werden. Der Rezeptor T1-M ist stets Membran-assoziiert und auf hämatopoetischen Zellen der Erythroid- und Mastzellinien konstitutiv vorhanden. Er repräsentiert 2 bis 5% des ingesamt erzeugten T1-Proteines (T1: T1-M und T1-S). Dagegen wird eine extrazelluläre lösliche Variante, die Liganden-bindende Domäne des vollständigen T1-M-Rezeptors, T1-S, in die Umgebung ausgeschieden. T1-S stellt jedoch kein Spaltprodukt von T1-M dar, sondern wird durch differentielles Spleißen von T1-RNA in differenzierenden Geweben einschließlich Knochen und Brustdrüsen exprimiert. T1-S stellt ca. 95% des erzeugten T1-Proteines dar; es wird in der neu gebildeten Knochenmatrix abgelagert. Die T1-S- und T1-M-Proteine sowie das für beide Proteine kodierende Gen sind ausführlich beschrieben in Klemenz (1989), Yanagisawa (1993) und Thomassen (1995) für Maus und in Tominaga (1992) für das menschliche Protein. Die Offenbarung dieser Literaturstellen ist hier ausdrücklich einbezogen.

T1-S wird in terminal differenzierten adulten Geweben nicht mehr gefunden (Rößler, 1995). Überraschenderweise haben die Erfinder nunmehr eine Tumorassoziierte Reexpression von T1-S in Osteosarkomen beobachtet.

Im folgenden wird ein kombiniertes in vitro/in vivo-Modell beschrieben, um die funktionelle Signifikanz einer strikt regulierten T1-Synthese während der Osteogenese zu demonstrieren. Eine klonale Osteosarkomzellinie, die nach subkutaner Injektion in syngenische Mäuse knochenähnliches Gewebe bilden kann (Schmidt, 1988) wurde mittels rekombinanter DNA-Methoden manipuliert, um konstitutiv erhöhte oder erniedrigte T1-Mengen zu exprimieren. Eine Verringerung der osteogenen Fähigkeit von klonalen homogenen Zellpopulationen in vitro hat eine im wesentlichen autokrine Funktion von T1 und osteogenen Zellen gezeigt, die auch in vivo offensichtlich war. Die Ergebnisse zeigen weiter den Wert dieses experimentellen Ansatzes bei der Unterscheidung zwischen dem inhärenten Potential von osteogenen Zellen und der Rolle von exogenen Faktoren, die durch die Wirtsumgebung bereitgestellt werden.

Frühere Arbeiten haben auf eine strikt regulierte Expression des T1-Genes bei der Osteogenese hingewiesen. Sie war gekennzeichnet durch eine hohe Expression von T1-mRNA und T1-Protein in frühen, proliferierenden Osteoblasten und der Repression der Expression in reifenden Zellen sowohl in vivo als auch in vitro. In allen bisher analysierten osteogenen Zellen stellte die Expression der 2,7 kb-mRNA, die für T1-S kodiert, 95 bis 99% der gesamten T1-spezifischen Transkripte dar, während die 5 kb-mRNA, deren Translation zum T1-M-Rezeptor führt, in weitaus geringeren Mengen vorhanden war (Rößler 1995, Werenskiold 1995). Die Erfinder haben nunmehr festgestellt, daß die Regulation der T1-Genexpression in osteogenen Zellen für die Initiation der Knochendifferenzierung in homogenen Zellpopulationen bzw. für die Stimulierung der Proliferation essentiell ist, und stellen erfindungsgemäß Verbindungen zur Verfügung, die die Aktivität des T1-M-Proteines modulieren können. "Modulation" bedeutet in diesem Zusammenhang eine durch die betreffende Verbindung hervorgerufene Veränderung der Aktivität und umfaßt sowohl deren Steigerung als auch Verringerung. Mit der Aktivität des T1-Proteines wird seine Fähigkeit bezeichnet, Differenzierungskompetenz zu verleihen.

Die erfindungsgemäße Zusammensetzung enthält gegebenenfalls neben der modulierenden Verbindung Zusatzstoffe, z.B. Stabilisatoren, Puffersubstanzen, osmotisch aktive Substanzen, etc.

Die Aktivität des T1-M-Proteines kann, wie hier gezeigt wird, durch Modulation auf Transkriptions- oder Translationsebene verändert werden. So kann beispielsweise die T1-M-Proteinaktivität auf Transkriptionsebene durch Bereitstellen zusätzlicher T1-M-mRNA gesteigert werden. Die Translation kann gestört werden, indem z.B. durch Bereitstellen von anti-sense-mRNA die gebildete sense-mRNA abgefangen wird und zu einer Translation nicht mehr zur Verfügung steht. Eine weitere Möglichkeit der Veränderung der Aktivität des Proteins ist jedoch auf der Ebene der Proteinfunktion selber, d.h. durch Wechselwirkung des T1-M-Proteines mit stimulierenden oder inhibierenden Verbindungen.

Die Beispiele zeigen, daß eine Herabsetzung der Aktivität des T1-M-Proteines, auf welchem Weg auch immer, die Proliferation der Knochenzellen steigert. Eine solche proliferationsfördernde modulierende Verbindung ist beispielsweise eine T1-anti-sense-Nukleinsäure (DNA oder RNA). So führt die Bereitstellung von anti-sense-mRNA in T1-M erzeugenden Zellen zur Inhibition der Translation der T1-mRNA. Folglich sinkt die Konzentration zur Verfügung stehender T1-M-Rezeptoren an der Zelloberfläche und die betroffene Zelle wird zur Proliferation angeregt.

Zur Inhibition der Translation bedarf es nicht unbedingt einer vollständigen T1-anti-sense-Nukleinsäure; erfahrungsgemäß ist es ausreichend, die Translation durch Hybridisierung mit einem Oligonukleotid, das komplementär zu einem Teil der T1-RNA ist, zu verhindern. Bevorzugte Oligonukleotide sind DNA-Oligonukleotide, die komplementär zum 5'-Bereich der T1-RNA sind.

In einer bevorzugten Ausführungsform ist die modulierende Verbindung ein DNA-Oligonukleotid, dessen Sequenz den Nukleotiden 191-1201 oder 277-796 der cDNA-Sequenz nach Klemenz, 1989, entspricht.

In einer weiteren bevorzugten Ausführungsform ist die modulierende Verbindung eine T1-S-sense-Nukleinsäure, z.B. eine T1-S-mRNA. Wie in den Beispielen gezeigt, führt die Expression von T1-S-mRNA zu einer Konkurrenz um den Liganden, wodurch das T1-M-Protein ebenfalls nicht aktiviert werden kann.

Eine weitere Ausführungsform sieht vor, den Rezeptor selbst zu inhibieren. Ein geeigneter Inhibitor ist beispielsweise ein monoklonaler und/oder ein polyklonaler Antikörper oder ein funktionelles Fragment eines solchen Antikörpers, das die Ligandenbindungsstelle blockiert. Verfahren zur Herstellung solcher Antikörper, sowohl polyklonaler als auch monoklonaler, sind im Stand der Technik gut bekannt. Sie setzen die Isolierung und weitgehende Reinigung des T1-Proteines voraus, die für das Maus-Protein bereits in Rupp, 1995, beschrieben ist. Nach Erhalt solcher Antikörper werden sie auf ihre inhibierende Fähigkeit getestet. Die Erzeugung von Antikörperfragmenten, z.B. Fab- oder F(ab)₂-Fragmenten, ist ebenfalls Stand der Technik.

Die Aktivität des membrangebundenen T1-M-Proteins kann weiterhin durch Kompetition um den Liganden erniedrigt werden. Modulierende Verbindungen, die geeignet sind, ein um den Liganden konkurrierendes Agenz bereitzustellen, sind z.B. Expressionsvektoren, die innerhalb der Zelle T1-S-mRNA exprimieren. Für eine Anwendung im menschlichen Patienten sind retrovirale Vektoren oder SV40-Vektoren bevorzugt. Die Konstruktion solcher Vektoren liegt im Bereich fachmännischen Könnens. Durch Einführen eines solchen Vektors in eine T1-M-produzierende Zelle wird somit indirekt ein kompetitiver Inhibitor bereitgestellt.

Kürzlich sind bindende Proteine beschrieben worden, die mögliche Liganden darstellen. In einer weiteren Ausführungsform der Erfindung wird das von Kumar (1995) beschriebene 18 kD-Protein aus BALB/c-3T3-Zellen als modulierende Verbindung eingesetzt.

Eine andere Möglichkeit ist die direkte Bereitstellung eines kompetitiven Inhibitors. Als solcher könnte beispielsweise das T1-S-Protein selber dienen. Wie oben bereits erwähnt, führt das Vorhandensein von T1-S-Protein zur Konkurrenz um den Liganden und damit zu einer fehlenden Aktivierung des T1-M-Proteines. Dadurch wird die Aktivität des T1-M-Proteines herabgesetzt und so die Proliferation von Knochenzellen stimuliert.

Zusammensetzungen, die die Aktivität des T1-M-Proteines erniedrigende modulierende Verbindungen enthalten, sind daher zur Behandlung von Osteoporosepatienten gut geeignet. Nach Applikation einer entsprechenden modulierenden Verbindung würde das Zellwachstum im behandelten Patienten zunächst beginnen und bei nachlassender Konzentration der modulierenden Verbindung später wieder eingestellt werden. Möglichkeiten, die Proliferation von stimulierten Knochenzellen zu verringern, ergeben sich auch durch den umgekehrten Weg, d.h. einer Steigerung der Aktivität des T1-M-Proteines.

Erfindungsgemäß werden auch Zusammensetzungen zur Verfügung gestellt, die der Steigerung der Aktivität des T1-M-Proteines dienen. Wie in den Beispielen gezeigt wird, geht mit einer gesteigerten Aktivität des T1-M-Proteines die Initiation der Differenzierung von Knochenzellen einher. Eine Steigerung der T1-M-Proteinaktivität kann z.B. durch Expressionsvektoren hervorgerufen werden, die für das T1-M-Protein kodieren. Durch eine erhöhte Kopienzahl des T1-M-Proteines wird, bis zur Erschöpfung des Liganden, die T1-M-Aktivität erhöht. Wie oben bereits erwähnt, eignen sich für die Verwendung in menschlichen Patienten in erster Linie SV40- oder retrovirale Vektoren.

In einer bevorzugten Ausführungsform ist die modulierende Verbindung ein Ligand für das T1-M-Protein. Kürzlich sind bindende Proteine beschrieben worden, die mögliche Liganden darstellen (Kumar 1995, Gayle 1996).

Die erfindungsgemäße Zusammensetzung zur Steigerung der Aktivität des T1-M-Proteines eignet sich zur Behandlung osteopetrotischer Erkrankungen.

Erfindungsgemäße Zusammensetzungen können oral, enteral, parenteral, subkutan, intramuskulär oder intraperitoneal verabreicht werden. Für die Applikation mittels Injektionen liegen sie in Form einer Lösung vor. Bevorzugt sind jedoch Applikationsformen, in denen die jeweilige modulierende Verbindung langsam freigesetzt wird, d.h. ein Depot der modulierenden Verbindung langsam abgebaut wird. Ein bevorzugtes Depotmaterial ist z.B. Matrigel® (Becton Dickinson, Heidelberg). Dem Fachmann sind weitere Depotmaterialien bekannt.

Die in den oben beschriebenen Zusammensetzung enthaltenen modulierenden Verbindungen werden erfindungsgemäß zur Herstellung von Medikamenten verwendet, die das Proliferations- und/oder Differenzierungspotential von Knochenzellen beeinflussen können. Dabei gilt das im Zusammenhang mit den Zusammensetzungen Ausgeführte entsprechend.

Erfindungsgemäß wird weiter ein Verfahren zur In vitro-Modulation der Aktivität des T1-M-Proteines zur Verfügung gestellt, bei dem Knochenzellen in Zellkultur einer die Aktivität des T1-M-Proteines modulierenden Verbindung ausgesetzt werden. In Abhängigkeit von der Wahl des modulierenden Mittels kann so in vitro eine Stimulation der Proliferation der Zellkultur oder eine Anregung der Differenzierung erreicht werden. Erfindungsgemäß in vitro zur Proliferation angeregte Knochenzellen könnten gegebenenfalls einem Osteoporosepatienten retransplantiert werden und so zur Milderung osteoporotischer Erscheinungen beitragen.

Die folgenden Abbildungen und die Beispiele erläutern die Erfindung, ohne sie einzuschränken.

### Beschreibung der Abbildungen

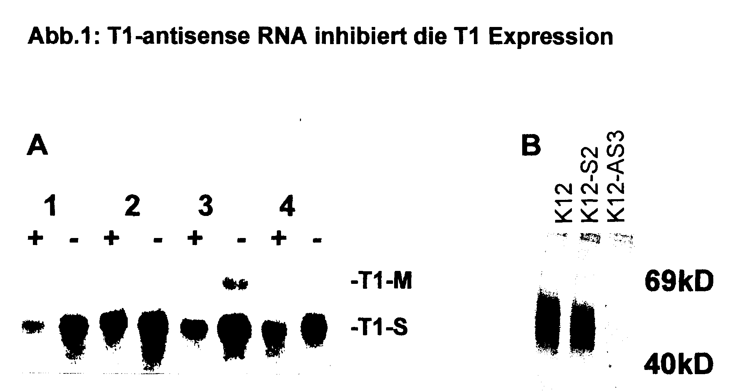

Abbildung 1: Die Modulation der T1-Synthese in Osteosarkomzellen durch T1-anti-sense-RNA.
   (A): Nachweis von T1-mRNA in Gesamt-RNA verschiedener K12-T1-anti-sense-Subklone mit durch das synthetische Glucocorticoid Dexamethason (DEX) induzierbarem anti-sense-Expressionsvektor in An- und Abwesenheit von DEX (50 nM) für 3 Stunden. 5 kb: T1-mRNA; 2,7 kb: T1-S-mRNA.
   (B): Immunpräzipitation von T1-S-Protein aus dem Medium von metabolisch markierten K12-, K12-S2- und K12-AS3-Osteosarkomzellen. Die Präzipitation wurde durchgeführt, wie in Werenskiold, 1992, beschrieben.

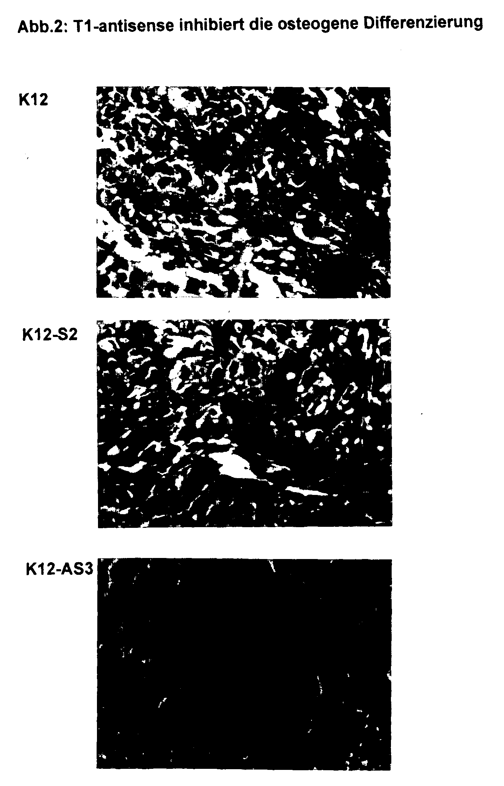

Abbildung 2: Schnitte der durch die in Abbildung 1B erwähnten Zellinien erzeugten, Formalin-fixierten und im Paraffin eingebetteten dekalzifizierten Tumore wurden gemäß van Giesson gefärbt, um die Bildung von Knochenmatrix sichtbar zu machen. K: Knochenmatrix; M: Mitose.

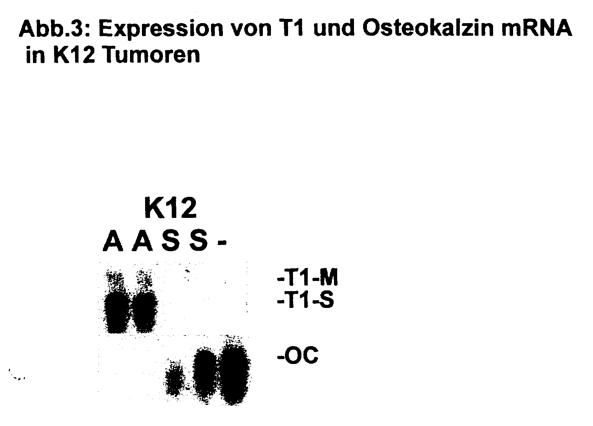

Abbildung 3: Tumor-assoziierte Expression von Osteokalzin- und T1-mRNAs. Die Gesamt-RNA aus gefrorenem Gewebe (Rößler 1995) von Tumoren von Parentalzellen (-), T1-sense-Zellen (S) und T1-anti-sense-Transfektanten (A) von K12-Zellen, wie angegeben, wurden mittels Northern Blot Hybridisierung auf Expression von Osteokalzin(OC - unten)- und T1-mRNA (T1 - oben) untersucht. Der T1-spezifische Northernblot zeigt 2 Banden: die obere T1-mRNA ist die 5 kb T1-Rezeptor-mRNA (T1-M), während die untere die 2,7 kb T1-S-mRNA (T1-S) darstellt (Werenskiold, 1995).

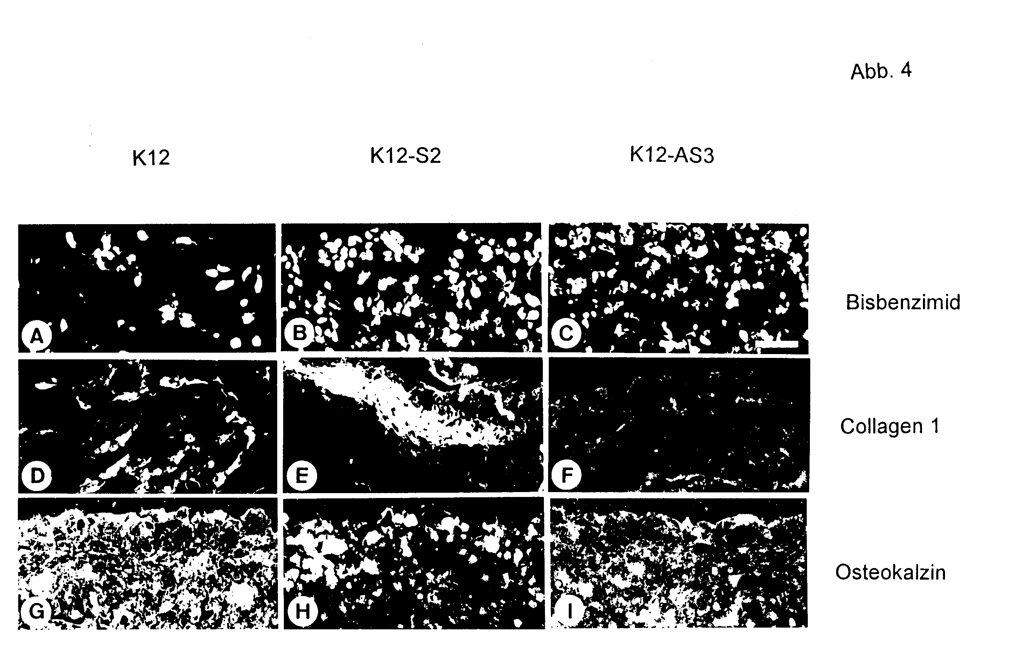

Abbildung 4: 3D-Kulturen von K12(A, D, G)-, K12-S2(B, E, H)- und K12-AS3(C, F, I)-Zellen wurden nach 30 Tagen Kultivierung in Stickstoff eingefroren; Gefrierschnitte der Kulturen wurden entweder mit Bisbenzimid (oben) (Kernfärbung) angefärbt, oder mit kommerziell erhältlichen Antikörpern gegen Collagen 1 (Mitte) bzw. Osteokalzin (unten).
Abbildung 5:
   (A): Differentielle Expression von Osteogenese-spezifischen mRNAs in 3-D-Kulturen von K12(-)-, K12-S2(S)- und K12-AS3(A)-Zellen sowie MC3T3-Zellen. Die Gesamt-RNA von je zwei individuellen Kulturen aller Zellinien wurde, wie in der Beschreibung zu Abb. 3 beschrieben, auf die Expression von T1-mRNAs (T1-M, T1-S), Collagen Typ 1 RNA (Coll1), alkalischer Phosphatase (AIP) und Osteokalzin (OC) analysiert.
   (B): Nachweis des sezernierten T1-S-Proteins in Medien der 3D-Kulturen von MC3T3-, K12-, K12-S2 und K12-AS3-Zellen.

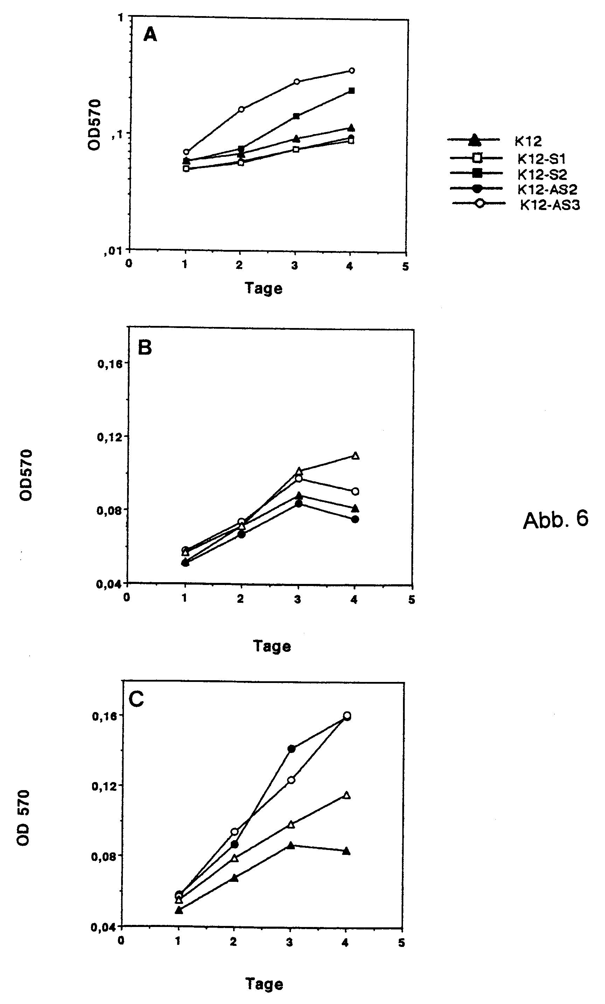

Abbildung 6: Einfluß von T1-anti-sense auf die Zellproliferation. Die Proliferation der verschiedenen Zellinien wurde mittels MTT-Test bestimmt.
   (A): Vergleich des Wachstums der Elterlinie K12 mit den Subklonen K12-AS2, K12-AS3, K12-S1 und K12-S2.
   (B) und (C): Einfluß von DEX auf das Wachstum von K12(B) und K12-AS2(C)-Zellen.
      - -O-:: Vorinkubation der Zellen für 3 Tage in Medium mit 50 nM DEX; Proliferationstest in Abwesenheit von DEX;
      - -O-:: Vorinkubation der Zellen für 3 Tage in Medium mit 50 nM DEX; Proliferationstest in Gegenwart von 50 nM DEX;
      - -△-: Vorinkubation und Proliferationstest in Abwesenheit von DEX;
      - -△-: Vorinkubation in Abwesenheit von DEX,
      Proliferation in Anwesenheit von 50 nM DEX.

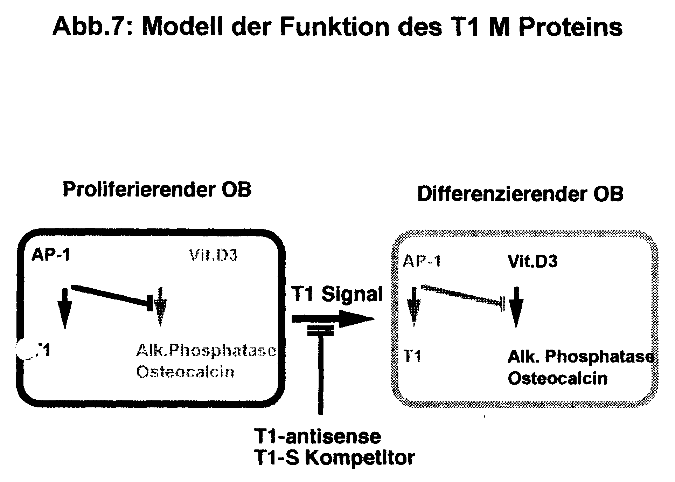

Abbildung 7: Modell der T1-Rezeptorfunktion bei der Knochendifferenzierung. In proliferierenden Osteoblasten (OB) treibt die hohe AP1-Aktivität die Expression des T1-Genes und reprimiert die Aktivierung der alkalischen Phosphatase- und Osteokalzingene durch Vitamin D₃. Nach Aktivierung des T1-Rezeptors wird die Zelle in reifende, nicht proliferierende Osteoblasten überführt, die durch eine niedrige AP1- und T1-Genaktivität und eine hohe Osteokalzin- und alkalische Phosphatase-Expression gekennzeichnet sind. Die T1-anti-sense-Expression und die Synthese von konkurrierendem T1-S blockieren die T1-Rezeptoraktivierung und arretieren die Zellen in einem undifferenzierten, proliferativen Zustand.

### Material und Methoden

### 1. Zellen und Zellkulturbedingungen

Die Etablierung der klonalen Zellinie K12, die von einem spontanen Osteosarkom einer BALB/c-Maus abgeleitet sind, sowie die Bedingungen für ihre Kultur sind bereits beschrieben worden (Schmidt, 1988).

Für dreidimensionale Kulturen wurden 5 x 10⁵ Zellen auf die obere Oberfläche von Scheiben (16 mm Durchmesser) aus einem dreidimensionalen Netzwerk aus Collagenfibern aufgebracht und wie beschrieben (Casser-Bette, 1990) kultiviert.

### 2. Proliferationstests

Für die Proliferationstests wurden 5 x 10⁵ Zellen pro Vertiefung mit 100 µl Medium in Mikrotiterplatten ausgesät. Es wurden Dreifachansätze gefahren. Zu den angegebenen Zeitpunkten wurden jeweils 10 µl MTT-PBS-Lösung (5 mg/ml) 3-(4,5-Dimethyl-thiazol-2-yl)-2,5-diphenyl-tetrazoliumbromid in phosphatgepufferter Kochsalzlösung) den Kulturen zugesetzt. Nach 4 Stunden Inkubation bei 37°C wurde die Reaktion durch Zusetzen von 100 µl 10 % Natriumdodecylsulfat in 10 mM HCl pro Vertiefung beendet und die Platten 15 Stunden bei 37°C inkubiert. Die MTT-Formazanerzeugung wurde durch Messen der OD bei 570 nm quantifiziert.

### 3. Konstruktion von Expressionsvektoren

Der Parentalvektor pLTR-stp (Jaggi et al., 1986) wurde mit XbaI und BamHI linearisiert. Ein 550 bp DNA-Fragment aus dem T1-ORF wurde unter Verwendung des Plasmides pT1.10 (Klemenz, 1989) mit dem folgenden Primerpaar amplifiziert: 5'-CGATCTAGAAGTAAATCGTCCTGGGGTC-OH und 5'-CGGGATCCCGGGCAGATCTAAGCTTTGTGAATGATCTGGTGGC-OH. Das resultierende PCR-Produkt wurde mit XbaI und BamHI geschnitten und in den linearisierten Vektor insertiert, was zum T1-anti-sense-Expressionsplasmid pLTR-T1as führte. Das vollständige T1-ORF (ca. 1 kb: Position 191-1201, Klemenz et al., 1989) wurde in sense-Orientierung in den Vektor pRc/CMV (Invitro-gen Corp., NL; Boshart et al., 1985) ligiert, um das Expressionsplasmid pCMV-T1-S zu ergeben.

### 4. RNA-Extraktion und -Analyse

Scheiben aus dreidimensionalen Kulturen wurden nach der Kulturperiode in Gegenwart von Guanidinisothiocyanatpuffer in flüssigem Stickstoff homogenisiert, um die RNA zu extrahieren.

Die RNA aus Einzelzellschichtkulturen, Tumorgewebe und Scheibenkulturen wurde gemäß Chirgwin et al., 1979, isoliert. Für die Northern Analyse wurden 8 bis 10 µg Gesamt-RNA glyoxyliert und in 1 %igen Agarosegelen aufgetrennt. Die verwendeten Hybridisierungssonden wurden aus Plasmiden abgeleitet, die cDNA-Sequenzen von T1 (Klemenz, 1989), Collagen 1 (Chu, 1982), Knochen-, Leber- und Nieren-spezifischer alkalischer Phosphatase (Weiss, 1986), Osteokalzin (Ford, 1985) und Glycerinaldehyd-3-phosphat-dehydrogenase enthielten.

### 5. Proteinanalyse

Die metabolische Markierung und Immunpräzipitation von T1-S wurde durchgeführt, wie bereits beschrieben (Werenskiold, 1992). Für den Nachweis von T1-S in 3 d-Kulturüberständen wurde 1 ml Medium 15 Stunden bei 40° in Gegenwart von 50 µl (Bettvolumen) Lentillektinsepharose (Sigma, München) inkubiert. Die Sepharoseperlen wurden 2 x mit PBS gewaschen und gebundene Proteine in SDS-Gelprobenpuffer solubilisiert. Die Western Bot Analyse der Proteinextrakte wurde durchgeführt, wie in (Werenskiold, 1992) beschrieben.

### Beispiel 1

### Untersuchung des Einflusses der T1-Genexpression auf die Differenzierung von Osteosarkomzellen

Der Einfluß der T1-Genexpression auf die Differenzierung von Osteosarkomzellen wurde in der osteogenen klonalen Tumorzellinie K12 untersucht. Die Zellinie ist von einem spontan aufgetretenen invasiven Osteosarkom einer BALB/c-Maus abgeleitet und induziert die Bildung von hoch differenzierten Osteosarkomen, sobald sie in syngenische Mäuse injiziert wird (Schmidt, 1988). Im Hinblick auf die T1-Genexpression verhält sich K12 wie eine typische Osteosarkomzelle: In K12-Zellen werden sowohl T1-mRNA als auch T1-Protein hoch exprimiert.

### Beispiel 2

### Modulation der T1-Genexpression in K12-Osteosarkomzellen

Zur Modulation der T1-Genexpression in K12-Zellen wurde T1-sense- bzw. T1-anti-sense-RNA in den Zellen erzeugt. Die Effizienz der T1-anti-sense-RNA vermittelten Inhibition der T1-mRNA-Expression wurde in einem induzierbaren System untersucht. Dazu wurden K12-Zellen mit einem Vektor (LTR-T1-AS; s.o.) transfiziert, indem das T1-ORF in anti-sense-Orientierung im Verhältnis zu dem Long Terminal Repeat des Maus-Mamma-Tumor-Virus, das Glucocorticoidresponsiv ist, insertiert war. Verschiedene neomycinresistente Transfektantenpools und -klone (K12-AS) synthetisierten T1-mRNA in Mengen, die der der Elterlinie K12 in Abwesenheit von Glucocorticoid vergleichbar waren. Die Behandlung von K12-AS-Zellen mit dem Glucocorticoid Dexamethason (DEX, 50 nM) reduzierte die Expression beider Varianten der T1-mRNA erheblich. Eine maximale Repression wurde in innerhalb von 3 Stunden nach Zusatz von DEX (Abbildung 1A) beobachtet und für mehrere Tage in Gegenwart des Hormones aufrecht erhalten. Um Zellinien mit permanent veränderten T1-Expressionsmustern zu erhalten, wurde das vollständige T1-S-ORF in sense-(CMV-T1-S)-Orientierung relativ zum konstitutiven Promotor des Cytomegalievirus (CMV) im Vektor pRc/CMV (s.o.) insertiert. Neomycinresistente Zellen, die nach Transfektion dieser Konstrukte in K12-Zellen erhalten wurden, wurden auf T1-mRNA-Synthese getestet (Daten nicht gezeigt). Das Durchmustern von K12-Transfektanten, die das LTR-T1-AS-Konstrukt enthielten, führte zur Isolierung der Sublinie K12-AS3, die konstitutiv niedrige T1-mRNA-Expression aufwies und auf den Zusatz von Hormonen nicht reagierte. Diese Zellinie wurde in weiteren Experimenten verwendet, um die konstitutive Repression der T1-mRNA-Synthese in K12-Zellen zu analysieren.

Die Immunpräzipitation des ausgeschiedenen T1-Proteines aus dem Überstand von metabolisch markierten Zellkulturen bestätigte die T1-Expressionsspiegel, die durch RNA-Analyse festgestellt worden war. Wie in Abbildung 1B gezeigt ist, haben K12- und K12-S2-Zellen große Mengen T1-Glykoprotein ausgeschieden, während nur geringe Mengen dieses Proteins aus dem Überstand von K12-AS3-Zellen präzipitiert werden konnten.

### Beispiel 3

### Die T1-Modulation interferiert mit der osteogenen Differenzierung von Tumoren

1 x 10⁶ Zellen der Zellinien K12, K12-S2, K12-AS3 wurden in 6 Wochen alte syngenische BALB/c-Mäuse injiziert. Das Tumorwachstum wurde beobachtet und die Mäuse wurden getötet, wenn die Tumore einen mittleren Durchmesser von 1 cm erreichten hatten.

Die histologische Untersuchung von Formalin-fixierten, in Paraffin eingebetteten Schnitten von Tumorgewebe nach H & E- oder van Giesson-Färbung zeigte eine Ossifikation der K12- und K12-S2-Tumore. Dagegen enthielten Tumore von K12-AS3-Zellen pleomorphe Zellen und eine hohe Zahl von Mitosen, aber wiesen keine Ossifikationsanzeichen auf (Abbildung 2).

Diese histologischen Ergebnisse wurden durch die Analyse der Genexpression in Gesamt-RNA bestätigt, die aus zwei Tumoren jeder Zellinie isoliert worden waren. Hohe Osteokalzin-mRNA-Spiegel waren in hoch differenzierten Tumoren von K12- und K12-S2-Zellen vorhanden, aber nicht nachweisbar in RNA der anaplastischen Tumore von K12-AS3-Zellen. Diese wiesen im Gegensatz zu hoch differenzierten Tumoren einen hohen T1-mRNA-Spiegel auf (Abbildung 3).

### Beispiel 4

### Einfluß der Deregulation der T1-Expression auf die osteogene Differenzierung in vitro

Die Kultivierung von osteogenen Zellinien über verlängerte Zeiträume innerhalb eines dreidimensionalen Netzwerks aus Collagen Typ 1 Fasern (3D-Kultur) gestattet die Differenzierung in vitro (Casser-Bette, 1996). Dieses Modell wurde verwendet, um die Fähigkeit von Tumorzellinien zur osteogenen Differenzierung in Abwesenheit von exogenen Faktoren zu bestimmen.

3D-Kulturen wurden mit den Zellinien K12 und ihren Sublinien K12-S2 und K12-AS3- und der nicht tumorigenen osteogenen Standard-Zellinie MC3T3 etabliert.

Die histologische Analyse von Gefrierschnitten der Kulturen zeigt starke Proliferation und mehrschichtiges Wachstum aller Zellinien (siehe Abb. 4, bis Benzimidfärbung). Nur in zellreichen Arealen von K12- und MC3T3-Zellen war Synthese und Ablagerung der differenzierungsspezifischen Proteine Collagen 1 und Osteokalzin nachweisbar.

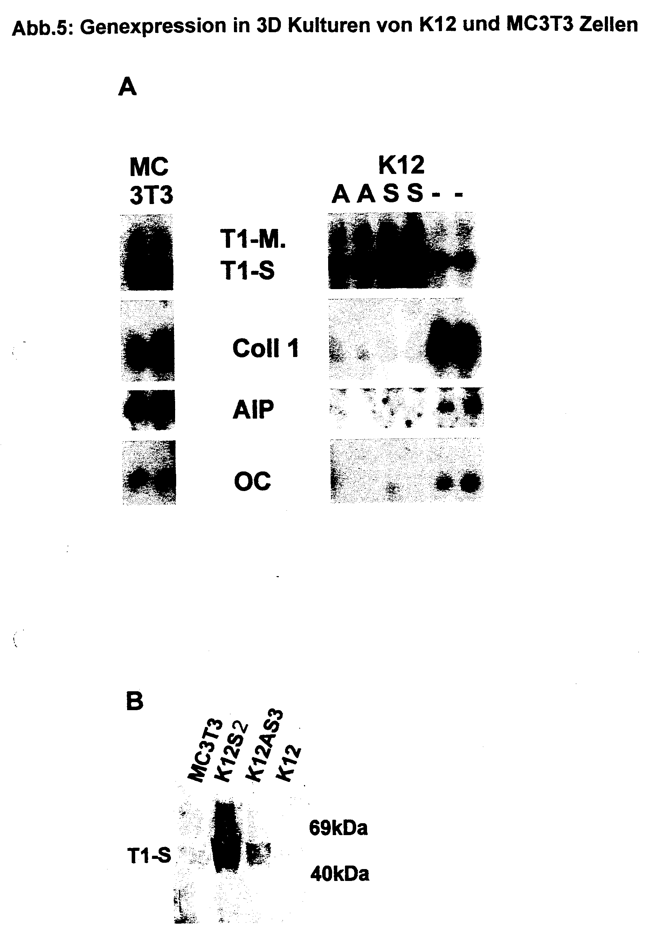

Der Nachweis der Expression Osteogenese-assoziierter Gene bestätigt diesen Befund: Die Differenzierung der 3D-Kulturen wurde durch Analyse der Expression von sequenziell exprimierten Osteogenese-assoziierten Genen nach 4 Wochen Kultur beobachtet. In den 3D-Kulturen von MC3T3 und K12-Zellen werden ähnliche Expressionsmuster für Osteogenese-spezifische Gene beobachtet. Collagen Typ 1 und T1-mRNAs, Marker der frühen (proliferativen) Phase der osteogenen Differenzierung, waren nur schwach exprimiert. Alkalische Phosphatase mRNA, die das zweite Stadium repräsentiert, war in K12-Kulturen in einem verglichen mit MC3T3-Kulturen verringerten Umfang vorhanden und Osteokalzin, ein Hauptexpressionsprodukt während der späten osteogenen Differenzierung, wurde in beiden Zellinien hoch exprimiert. In K12-S2- und K12-AS3-Kulturen konnte keine für alkalische Phosphatase kodierende mRNA nachgewiesen werden, und die mRNAs für Typ 1 Collagen und Osteokalzin waren nur schwach sichtbar. Im Gegensatz dazu war endogene T1-mRNA in K12-S2-Zellen vorherrschend und in einem geringen Umfang auch in K12-AS3-Zellen vorhanden (Abbildung 5A).

Die Akkumulation von T1-S-Protein im Kulturmedium am Ende des 3D-Kulturzeitraums wurde mittels Western Blot Analyse überprüft. Das Medium von K12-S2 enthielt einen hohen Spiegel T1-S-Protein und das Medium von K12-AS3- und MC3T3-Zellen einen niedrigen, was die Menge der in den entsprechenden 3D-Kulturen vorhandenen mRNA reflektiert (Abbildung 5B).

### Beispiel 5

### T1-anti-sense vermittelt eine gesteigerte Proliferation von K12-Zellen

Um die Wachstumskinetiken verschiedener K12-Sublinien mit denen der Elterlinie zu vergleichen, wurden 1 x 10³ Zellen pro 0,1 ml in 4-fach-Ansätzen in Mikrotiterplatten ausgesät und die Proliferation in 24-Stunden-Intervallen 4 Tage unter Verwendung eines standardisierten MTT-Tests (Boehringer Mannheim, FRG) beobachtet. Die meisten K12-Sublinien (einschließlich der induzierbaren LTR-T1-AS-Linien) zeigten Proliferationsraten, die denen der Elterzellen vergleichbar waren (K12-S1, K12-AS2) oder etwas höher (K12-S2) (Abbildung 6A). K12-AS3-Zellen, gekennzeichnet durch eine konstitutiv niedrige T1-Expression, wuchsen signifikant schneller als die Mehrzahl der Klone.

Eine mögliche Rolle der erniedrigten T1-Synthese bei der gesteigerten Proliferation von K12-AS3-Zellen wurde in einer Zellinie untersucht, die ein DEX-induzierbares T1-anti-sense-Konstrukt (K12-AS2) enthielt. K12, K12-S2 und K12-AS2 wurden 3 Tage in Gegenwart oder Abwesenheit von 50 mM DEX vorkultiviert und anschließend in Mikrotiterplatten ausgesät. Die Proliferation der Zellen in Gegenwart oder Abwesenheit von 50 nM DEX wurde getestet, wie oben beschrieben. Sowohl K12 als auch K12-S2-Zellen wuchsen 3 Tage mit ähnlichen Kinetiken. Die Gegenwart von DEX während der Vorkultivierung und/oder während der Testzeitraums verringerte die Zelldichte nach 4 Tagen Kultur (Abbildung 6B). Im Gegensatz dazu steigerte die Vorkultivierung von K12-AS2-Zellen in Gegenwart von 50 nM DEX ihre Proliferation sowohl in Anwesenheit als auch in Abwesenheit von DEX während des folgenden Testzeitraums signifikant. Der Zusatz von DEX nur während des Testzeitraumes steigerte die letztendliche Zelldichte geringfügig (Abbildung 6C).

### Zusammenfassende Betrachtung der Ergebnisse

Frühere Arbeiten haben eine strikt regulierte Expression des T1-Genes bei der Osteogenese gezeigt. Sie war durch eine hohe Expression von T1-mRNA und T1-Protein in frühen proliferierenden Osteoblasten und Repression der reifenden Zelle sowohl in vivo als auch in vitro gekennzeichnet. In allen bisher analysierten osteogenen Zellen stellte die 2,7 kb-T1-S-mRNA 95 bis 99% der T1-spezifischen Transkripte dar, während die 5 kb mRNA, die für den T1-M-Rezeptor kodiert, nur in geringem Umfang vorhanden war (Werenskiold 1995, Rößler 1995). Die oben beschriebenen Experimente zeigen, daß die strikte Regulation der T1-Genexpression in osteogenen Zellen für die Initiation der Knochendifferenzierung essentiell ist.

Es konnte gezeigt werden, daß die gut charakterisierte klonale Maus-Osteosarkomzellinie K12 in vivo und überraschenderweise in vitro osteogen differenzieren kann. Das etablierte 3D-Kultursystem erlaubt eine osteogene Differenzierung von K12-Zellen in einem ähnlichen Ausmaß wie bei der gut charakterisierten, nicht tumorigenen osteogenen Zellinie MG3T3. K12-Zellen waren daher zur terminalen Differenzierung in Gegenwart einiger exogener Faktoren, die durch die Mikroumgebung des Wirtes bereitgestellt wurden, fähig. Sie stellen daher ein geeignetes Modell zur Untersuchung der Effekte der modulierten T1-Expression sowohl in homogenen Zellpopulation in Abwesenheit von exogenen Einflüssen in vitro als auch nach Injektion in syngenische Tiere in vivo dar. Die konstitutive Expression von T1-sense- und T1-anti-sense-RNA erwies sich als wirksam zur Modulation der T1-Expressionsmuster in Osteosarkomzellen.

Infolge der Expression von zwei T1-Proteinvarianten müssen verschiedene Ebenen der Wechselwirkung der T1-Modulation mit T1-Funktionen in Betracht gezogen werden:
1. T1-S-sense-RNA erzwingt die konstitutive Produktion des ausgeschiedenen T1-Glykoproteins. Wenn es in ausreichend hoher Menge in der zellulären Umgebung vorhanden ist, wirkt dieses Protein als ein konstitutiver Inhibitor des T1-M-Rezeptors.
2. T1-anti-sense-RNA inhibiert die Synthese sowohl des seltenen T1-M-Rezeptors (kodiert von der 5 kb-mRNA) als auch des reichlich vorhandenen sekretorischen T1-S-Proteines (kodiert von der 2,7 kb-mRNA) (Abbildung 1). Die anti-sense-vermittelte Inhibition der T1-Ausscheidung ist komplementär zur Wirkung des T1-sense-Konstruktes. Die Inhibition der T1-M-Rezeptorsynthese wirkt jedoch auf einer anderen Ebene und kann direkt mit der T1-Rezeptorsignalkette in T1-anti-sense exprimierenden Zellen interferieren. All diese Ebenen der Wechselwirkung mit der Zellfunktion sind offensichtlich in die oben beschriebenen Ergebnisse involviert.

### T1-anti-sense-Wirkungen

Die Inhibition der T1-Synthese beeinflußt Wachstum und Differenzierung der K12-Zellinie. K12-AS-Zellen wuchsen schneller in Kultur. In Zellen mit induzierbarer T1-anti-sense-Expression erfordert die Wachstumssteigerung zunächst eine erniedrigte T1-Synthese vor der Analyse der Proliferation. Dieses Ergebnis steht in scharfem Kontrast zu der beobachteten schnellen und wirksamen antisense-induzierten Inhibition der T1-S-Synthese. Es weist darauf hin, daß es eher die erniedrigte Expression der zellulären T1-M-Rezeptors als die des ausgeschiedenen T1-S-Proteines ist, die bei den Zellen eine schnellere Proliferation bewirkt.

Weiterhin hebt T1-anti-sense die osteogenen Fähigkeiten von K12-Zellen in vitro und in vivo auf. Dieser Differenzierungsblock kann in Gegenwart von exogenen Faktoren, die die terminale Differenzierung von Elter-K12-Zellen stimulierten, nicht überwunden werden. Die T1-anti-sense-vermittelte Inhibition der osteogenen Differenzierung ist daher ein inhärenter Defekt von K12-AS-Zellen, für den höchstwahrscheinlich der T1-M-Rezeptor verantwortlich ist.

### T1-sense-Effekte

Die konstitutive Synthese von T1-S in K12-S-Zellen zeigte differentielle Wirkungen in den experimentellen Systemen. In proliferierenden Einzelschichtkulturen, in denen das ausgeschiedene Protein im Überstand verdünnt wird, wird kein Effekt beobachtet. In 3D-Kulturen inhibiert jedoch eine konstitutive T1-S-Ausscheidung die Osteogenese wirksam. Dies kann eine Folge einer lokal erhöhten Konzentration von sezerniertem T1-S in einer differenzierungsfreundlichen Mikroumgebung infolge einer hohen lokalen Zelldichte sein. In experimentellen Tumoren wird die inhibitorische Aktivität des konstitutiv sezernierten T1-S überwunden, wahrscheinlich durch seine Aufnahme in Wirtsgewebe.

### T1-Funktion in osteogenen Zellen

Die mit K12-Zellen erhaltenen Ergebnisse stehen mit der folgenden Interpretation im Einklang: K12-Zellen wurden in einem frühen, differenzierungskompetenten Zustand immortalisiert, der durch eine hohe T1-Genaktivität charakterisiert ist. Osteogene Zellen im frühen proliferativen Stadium der Differenzierung exprimieren sowohl sekretorisches T1-S als auch den T1-M-Rezeptor. Die Inhibition der T1-Rezeptorsignalkette durch T1-anti-sense-RNA in diesen Zellen blokkierte die osteogene Differenzierung in diesem frühen Stadium vollständig, während sie die Proliferation in unreifen Zellen steigerte. Dieses Ergebnis läßt vermuten, daß ein T1-Rezeptor-vermitteltes Signal notwendig ist, um einen differenzierungskompetenten osteogenen Zustand aufrecht zu erhalten (begleitet von einer niedrigeren Proliferationsrate). Eine osteogene Differenzierung wurde auch durch Stören der T1-Rezeptorsignalkette durch konkurrierendes extrazelluläres T1-S-Protein inhibiert. Die T1-S-vermittelte Inhibition war jedoch für experimentelle Bedingungen empfindlicher, was auf das Erfordernis hoher lokaler Proteinkonzentrationen schließen läßt.

Auf molekularer Ebene spiegelte sich die Inhibition der Differenzierung in der Abwesenheit osteogenesespezifischer Transkripte, die für alkalische Phosphatase und Osteokalzin kodieren, wider. Tatsächlich unterstützen sowohl die 3D-Kulturen als auch die Tumore die Annahme, daß die Expression von T1 und Osteokalzingenen sich gegenseitig ausschließen. Eine mögliche Erklärung für dieses Phänomen liegt in der Promotorstruktur der responsiven Gene. Die T1-Genexpression wird durch den fos-jun-Transkriptionsfaktor (AP-1, Trüb 1995) aktiviert, während die Vitamin D abhängige Aktivierung des Osteokalzingenpromotors durch Binden von AP-1-Komplexen mit den überlappenden Vitamin D-responsiven Element blockiert werden kann (Schüle 1995, Owen 1990, 1993). Da die Störung der T1-Rezeptorsignalkette eine verlängerte Zellproliferation fördert und damit die andauernde Gegenwart des fos-Transkriptionsfaktors, bleiben die Vitamin D abhängigen Promtoren von differenzierungsspezifischen Genen blockiert.

Zusammengefaßt schlagen die Erfinder vor, daß der T1-M-Rezeptor in unreifen osteogenen Zellen ein essentielles Signal für die Etablierung eines differenzierungskompetenten Zustands, der mit geringer Proliferation verbunden ist, gibt. Die Inhibition seiner Funktion steigert das Zellwachstum auf Kosten der Differenzierungskompetenz. Diese Interpretation impliziert eine autokrine Erzeugung des T1-Liganden in unreifen osteogenen Zellen. Die Demonstration eines möglichen T1-Liganden in 3T3-Fibroblasten, die ein ähnliches T1-Expressionsmuster aufweisen, stützt diese Vermutung.

### Literaturliste

Boshart et al., 1985, Cell 41, 521-530.
Casser-Bette, M.; Murray, A. B.; Closs, E. I.; Erfle, V., and Schmidt, J. (1990) Calcif. Tissue Int., 46, 46-56.
Chirgwin, J. M.; Przybyla, A. E.; McDonald, R. J., and Rutter, W. J. (1979) Biochemistry, 18, 5294-5299.
Chu, M. L.; Myers, J. C.; Bernard, M. P.; Ding, J. F., and Ramirez, F. (1982) Nuc. Acids Res., 10, 5925-5934.
Fort, P.; Piechaczyk, M.; El Sabrouty, S.; Dani, C.; Jeanteur, P., and Blanchard, J. M. (1985) Nuc. Acids Res., 13, 327-334.
Gayle, M. A.; Slack, J. L.; Bonnert, T. P.; Renshaw, B. R.; Sonoda, G.; Taguchi, T.; Testa, J. R.; Dower, S. K., and Sims, J. E. (1996) J. Biol. Chem., 271, 5784-5789.
Jaggi, R., Salmons, B., Müller, R. und Groner, B., 1986, EMBO J 5, 2609-2616
Klemenz, R.; Hoffmann, S., and Werenskiold, A. K. (1989) Proc. Natl. Acad. Sci. USA, 86, 5708-5712.
Kumar, S.; Minnich, M. D., and Young, P. R. (1995) J. Biol. Chem., 270, 27905-27913.
Owen, T. A.; Aronow, M.; Shalhoub, V.; Barone, L. M.; Wilming, L.; Tassinari, M. S.; Kennedy, M. B.; Pockwinse, S.; Lian, J. B., and Stein, G. S. (1990) J. Cell. Physiol., 143, 420-430.
Owen, T. A.; Bortell, R.; Shaloub, V.; Heinrichs, A.; Szein, J. A.; Stein, G. S., and Lian, J. B. (1993) Proc. Natl. Acad. Sci. USA, 90, 1503-1507.
Rößler, U.; Thomassen, E.; Hültner, L.; Baier, S.; Danescu, J., and Werenskiold, A. K. (1995) Dev. Biol, 168, 86-97.
Rupp, B.; Rößler, U.; Löwel, M., and Werenskiold, A. K. (1995) Biochem. Biophys. Res. Com., 216, 595-601.
Schmidt, J.; Strauß, P. G.; Schön, A.; Luz, A.; Murray, A. B.; Melchiori, A.; Aresu, O., and Erfle, V. (1988) Differentiation, 39, 151-160.
Schüle, R.; Umesono, K.; Mangelsdorf, D.J.; Bolado, J. and Evans, M. (1990) Cell, 61, 497-504.
Thomassen, E.; Kothny, G.; Haas, S.; Danescu, J.; Hültner, L.; Dörmer, P., and Werenskiold, A. K. (1995) Cell Growth Differ., 6, 178-184.
Tominaga, S.; Yokota, T.; Yanagisawa, K.; Tsukamoto, T.; Takagi, T., and Tetsuka, T. (1992) Biochim. Biophys. Acta, 1171, 215-218.
Trueb, T.; Kalousek, M. B.; Fröhli, E., and Klemenz, R. (1994) Proc. Natl. Acad. Sci. USA, 91, 3896-3900.
Weiss, M. J.; Henthorn. P.S.; Lafferty, M. A.; Slaughter, C.; Raducha, M., and Harris, H. (1986) Proc. Natl. Acad. Sci. USA, 83, 7182-7186.
Werenskiold, A. K. (1992) Eur. J. Biochem., 204, 1041-1047.
Werenskiold, A. K.; Rößler, U.; Löwel, M.; Schmidt, J.; Heermeier, K.; Spanner, M., and Strauss, P. G. (1995) Cell Growth Differ., 6, 171-177.
Yanagisawa, K.; Takagi, T.; Tsukamoto, T.; Tetsuka, T., and Tominaga, S. (1993) FEBS Lett., 318, 83-87.

## Patentansprüche

1. Zusammensetzung, dadurch gekennzeichnet, daß sie eine Verbindung enthält, die die Aktivität des endogenen T1-M-Proteines direkt oder indirekt modulieren kann, sowie gegebenenfalls physiologisch verträgliche Zusatzmittel.

2. Zusammensetzung nach Anspruch 1, dadurch gekennzeichnet, daß die modulierende Verbindung die Aktivität des T1-M-Proteines durch Modulation der Expression beeinflußt.

3. Zusammensetzung nach Anspruch 1, dadurch gekennzeichnet, daß die modulierende Verbindung die Aktivität des T1-M-Proteines durch Wechselwirkung mit diesem beeinflußt.

4. Zusammensetzung nach einem der Ansprüche 1 bis 3, dadurch gekennzeichnet, daß sie die Aktivität des T1-M-Proteines herabsetzt.

5. Zusammensetzung nach einem der Ansprüche 1, 2 oder 4, dadurch gekennzeichnet, daß die modulierende Verbindung eine T1-anti-sense-Nukleinsäure ist.

6. Zusammensetzung nach Anspruch 5, dadurch gekennzeichnet, daß die modulierende Verbindung ein Oligonukleotid ist, das komplementär zu einem Teil der T1-mRNA ist.

7. Zusammensetzung nach Anspruch 6, dadurch gekennzeichnet, daß das Oligonukleotid komplementär zum 5'-Bereich des T1-ORFs ist.

8. Zusammensetzung nach Anspruch 7, dadurch gekennzeichnet, daß die modulierende Verbindung ein DNA-Oligonukleotid mit der Sequenz der Nukleotide 191-1201 oder 277-796 der cDNA-Sequenz ist.

9. Zusammensetzung nach einem der Ansprüche 1, 2 oder 4, dadurch gekennzeichnet, daß die modulierende Verbindung eine T1-S-sense-Nukleinsäure ist.

10. Zusammensetzung nach Anspruch 9, dadurch gekennzeichnet, daß die modulierende Verbindung eine T1-S-mRNA ist.

11. Zusammensetzung nach Anspruch 3, dadurch gekennzeichnet, daß die modulierende Verbindung ein Inhibitor des Rezeptors ist.

12. Zusammensetzung nach Anspruch 11, dadurch gekennzeichnet, daß der Inhibitor ein monoklonaler und/oder polyklonaler Antikörper oder ein funktionelles Fragment eines solchen ist.

13. Zusammensetzung nach Anspruch 11, dadurch gekennzeichnet, daß die modulierende Verbindung die Aktivität des T1-M-Proteins durch Kompetition um den Liganden beeinflußt.

14. Zusammensetzung nach Anspruch 13, dadurch gekennzeichnet, daß die modulierende Verbindung ein Expressionsvektor ist, der zur Expression von T1-S fähig ist.

15. Zusammensetzung nach Anspruch 13, dadurch gekennzeichnet, daß die modulierende Verbindung ein kompetitiver Inhibitor ist.

16. Zusammensetzung nach Anspruch 15, dadurch gekennzeichnet, daß der kompetitive Inhibitor T1-S-Protein ist.

17. Zusammensetzung nach einem der Ansprüche 4 bis 16, dadurch gekennzeichnet, daß sie zur Behandlung von Osteoporosepatienten geeignet ist.

18. Zusammensetzung nach einem der Ansprüche 1 bis 3, dadurch gekennzeichnet, daß sie die Aktivität des T1-M-Proteines erhöht.

19. Zusammensetzung nach Anspruch 18, dadurch gekennzeichnet, daß die modulierende Verbindung ein Expressionsvektor ist, der zur Expression von T1-M fähig ist.

20. Zusammensetzung nach Anspruch 18 oder 19, dadurch gekennzeichnet, daß sie die Differenzierung von Knochenzellen anregt.

21. Zusammensetzung nach einem der Ansprüche 18 oder 20, dadurch gekennzeichnet, daß die modulierende Verbindung ein T1-Ligand ist.

22. Zusammensetzung nach Anspruch 21, dadurch gekennzeichnet, daß der Ligand ein 18 kd-Protein als BALB/c-3T3-Zellen ist.

23. Zusammensetzung nach einem der Ansprüche 18 bis 22, dadurch gekennzeichnet, daß das Medikament zur Behandlung osteopetrotischer Erkrankungen geeignet ist.

24. Zusammensetzung nach einem der Ansprüche 1 bis 23, dadurch gekennzeichnet, daß sie zur oralen, enteralen, parenteralen, subkutanen, intramuskulären oder intraperitonealen Applikation geeignet ist.

25. Zusammensetzung nach Anspruch 24, dadurch gekennzeichnet, daß sie in Form einer Lösung vorliegt.

26. Zusammensetzung nach Anspruch 25, dadurch gekennzeichnet, daß sie als Depot zubereitet ist.

27. Verwendung von Verbindungen, die die Aktivität von T1-M-Protein modulieren, zum Herstellen eines Medikamentes zum Beeinflussen des Proliferations- und/oder Differenzierungspotentials von Knochenzellen.

28. Verwendung nach Anspruch 27, dadurch gekennzeichnet, daß die Aktivität des T1-M-Proteins durch Modulation der Expression beeinflußt wird.

29. Verwendung nach Anspruch 26, dadurch gekennzeichnet, daß die Aktivität des T1-M-Proteins durch Wechselwirkung des T1-M-Proteines mit einer modulierenden Verbindung beeinflußt wird.

30. Verwendung nach einem der Ansprüche 26 bis 28, dadurch gekennzeichnet, daß die Aktivität herabgesetzt wird.

31. Verwendung nach einem der Ansprüche 26, 27 oder 29, dadurch gekennzeichnet, daß die modulierende Verbindung eine T1-anti-sense-Nukleinsäure ist.

32. Verwendung nach einem der Ansprüche 26, 27 oder 29, dadurch gekennzeichnet, daß die modulierende Verbindung eine T1-S-sense-Nukleinsäure ist.

33. Verwendung nach Anspruch 28, dadurch gekennzeichnet, daß die modulierende Verbindung ein Inhibitor des Rezeptors ist.

34. Verwendung nach Anspruch 32, dadurch gekennzeichnet, daß der Inhibitor ein monoklonaler und/oder polyklonaler Antikörper oder ein funktionelles Fragment eines solchen ist.

35. Verwendung nach Anspruch 32, dadurch gekennzeichnet, daß die modulierende Verbindung ein kompetitiver Inhibitor ist.

36. Verwendung nach Anspruch 34, dadurch gekennzeichnet, daß der kompetitive Inhibitor T1-S-Protein ist.

37. Verwendung nach einem der Ansprüche 29 bis 35, dadurch gekennzeichnet, daß das Medikament zur Behandlung von Osteoporosepatienten geeignet ist.

38. Verwendung nach einem der Ansprüche 25 bis 28, dadurch gekennzeichnet, daß die Aktivität erhöht wird.

39. Verwendung nach Anspruch 37, dadurch gekennzeichnet, daß die Differenzierung von Knochenzellen angeregt wird.

40. Verwendung nach einem der Ansprüche 37 oder 38, dadurch gekennzeichnet, daß die modulierende Verbindung ein T1-Ligand ist.

41. Verwendung nach Anspruch 39, dadurch gekennzeichnet, daß der Ligand ein 18 kD-Protein aus BALB/c-3T3-Zellen ist.

42. Verwendung nach einem der Ansprüche 37 bis 40, dadurch gekennzeichnet, daß das Medikament zur Behandlung osteopetrotischer Erkrankungen geeignet ist.

43. Verfahren zur in vitro-Modulation der Aktivität des T1-M-Proteines, dadurch gekennzeichnet, daß Zellkulturen einer die Aktivität des T1-M-Proteines modulierenden Verbindung ausgesetzt werden.

44. Verfahren nach Anspruch 42, dadurch gekennzeichnet, daß die Aktivität herabgesetzt wird, wodurch die Proliferation der Knochenzellen angeregt wird.

45. Verfahren nach Anspruch 42, dadurch gekennzeichnet, daß die Aktivität erhöht wird, wodurch die Knochenzellen zur Differenzierung angeregt werden.
